Europäisches Patentamt

European Patent Office

Office européen des brevets

(11) Publication number: **0 364 173**
**A1**

## (12) EUROPEAN PATENT APPLICATION

(21) Application number: **89310258.2**

(22) Date of filing: **06.10.89**

(51) Int. Cl.5: **B01D 29/01 , B01D 63/08 , A61M 1/36**

(30) Priority: **10.10.88 GB 8823706**

(43) Date of publication of application:
**18.04.90 Bulletin 90/16**

(84) Designated Contracting States:
**BE CH DE ES FR GB GR IT LI NL SE**

(71) Applicant: **ALCAN INTERNATIONAL LIMITED**
**1188 Sherbrooke Street West**
**Montreal Quebec H3A 3G2(CA)**

(72) Inventor: **Philpott, Richard William**
**Lamb Cottage 4 Thorpe Mandeville Road**
**Upper Wardington Banbury Oxon OX17**
**1SR(GB)**

(74) Representative: **Pennant, Pyers et al**
**Stevens, Hewlett & Perkins 5 Quality Court**
**Chancery Lane**
**London, WC2A 1HZ(GB)**

(54) Microfilter device.

(57) A micro-filter device, suitable for removing micro-organisms such as mycoplasma from biological fluids comprises two filters in series. The first is a pre-filter. The second is a porous asymmetric inorganic membrane, in which the pores adjacent one face are larger diameter than those adjacent the other face, the large-pore face being positioned facing upstream.

EP 0 364 173 A1

## MICROFILTER DEVICE

This invention relates to microfilter devices. The term microfilter is used to indicate that the pores of the filter are of sub-micron size. Microfilters, based both on organic polymers and on inorganic materials, are commercially available. Inorganic microfilters have advantages over organic mircofilters, of better resistance to various chemicals, better heat stability which may be important for e.g. sterilizing purposes, and often better control over pore size and higher flux for a given pore size. This invention is concerned with inorganic microfilters and not with organic ones.

Filter design generally involves compromising between two important features:
- pore size, which determines the minimum size of suspended particulate material that can be retained by the filter; and
- flux, the rate of flow per unit area of filter under defined conditions. Naturally, flux varies inversely with pore size. But flux also varies inversely with pore length, and it is therefore generally desired to make the narrow pores as short as possible, i.e. to make the narrow pore portion of the filter as thin as possible.

Because very thin membranes are fragile, many people have attempted to design composite membranes in which a thin microporous film is supported on a thicker more robust porous membrane. Provided that the pores of the porous membrane are not too large, the applied microporous film can bridge them without cracking in normal use. The result is an asymmetric membrane. There are many examples of asymmetric inorganic membranes in the literature, including the following: EPA 040282; EPA 136937; EPA 144097; EPA 178831; EPA 188950; EPA 242208; USP 4562021; USP 4689150. These asymmetric membranes have a system of larger pores extending in from one face and interconnecting with a system of smaller pores extending in from the other face. Based on the idea that the smaller pore region is supported by the larger pore region, these asymmetric membranes are used as filters, always with the smaller pore region on the upstream side.

US 4629563 describes asymmetric microporous membranes of organic polymer with a skin side and a support side, and teaches use of the membranes as filters with the support side facing upstream.

Anotec Separations markets, under the trade mark ANOTOP, a range of disposable syringe filters including an asymmetric inorganic filter membrane. While these have been generally successful, there is still room for improvement, both in flux and filtering efficiency, and in the amount of liquid which can be filtered before the filter blocks.

In one aspect this invention provides a microfilter device comprising a housing having entry and exit ports and defining a passage for fluid and extending across said passage two filters in series, wherein:
- the first filter is a pre-filter
- the second filter is an inorganic membrane having two faces connected by pores, the diameter of the pores adjacent a first face being larger than the diameter of the pores adjacent a second face, the first face being positioned on the upstream side of the membrane.

In another aspect, the invention provides a method of treating a liquid containing suspended material, which method comprises passing the liquid through the microfilter device herein described.

This invention results from the discovery that two features in combination give rise to unexpected improved filter performance in various directions. The first feature is the use of a pre-filter, whose function is to remove coarser particulate material that might otherwise clog the main filter. The pore size of the pre-filter is therefore made somewhat larger than that of the main filter. The nature of the pre-filter is not critical, and it has been found convenient to use a filter having a thickness of from 0.2 to 5 mm preferably from 0.4 to 2 mm with a pore size of 0.2 to 5 $\mu$m preferably 0.4 to 1 $\mu$m. The pre-filter may be organic or inorganic or metallic. Tortuous path filters such as those of glass fibre are suitable.

The second feature is the use of an inorganic asymmetric membrane with the smaller pore region facing downstream rather than upstream. The asymmetric membrane is preferably an anodic aluminium oxide membrane, such as may be prepared by the technique described in EPA 178831 mentioned above. An anodic aluminium oxide membrane according to that specification has a system of larger pores extending in from one face and interconnecting with a system of smaller pores extending in from the other face. The membrane is made by anodising an aluminium metal substrate, then reducing the applied voltage at a rate to permit partial or complete recovery of the oxide membrane, either continuously or incrementally in small steps down to a level preferably below 15V and separating the oxide membrane from the substrate. Asymmetric membranes of this kind are commercially available under the trade mark ANODISC. The pores in the system of smaller pores typically have a minimum diameter of at least 2 nm but less than half that of the system of larger pores, for example in the range 5 to 150 nm. For use in the present invention, the minimum pore size is preferably in the range 30 to 120 nm.

Alternatively, the second filter may be an anodic aluminium oxide membrane which is asymmetric by virtue of the fact that the pores which extend through it are tapered, their diameter adjacent the first face being larger than adjacent the second face.

Alternatively, the second filter may be a composite membrane comprising a porous inorganic membrane, e.g. anodic or tape-cast, and a microporous inorganic film, e.g. produced by a sol-gel technique, overlying one face thereof. Such composite membranes are described in various patents noted above, including particularly EPA 242208.

Both the first and second filters are preferably inorganic and non-metallic. Either or both filters may be supported, either round their periphery or across their entire area, in conventional manner. For example, as in current ANOTOP filter devices, a dimpled porous support of plastic material may be used, with the filter bonded to the tops of the dimples. The two filters may be kept separate within the housing, with a space for fluid between them; or the first filter may simply rest, with or without spacer means, on the upstream surface of the second filter. The housing preferably constitutes a hand-held syringe filter device, which may be made disposable. To this end, the entry port may be adapted, e.g. by means of a luer lock, to receive fluid from a hand-operated syringe; and the outlet port may be adapted to receive a hypodermic needle. The housing may be made of a plastics material such as polypropylene. In an alternative arrangement a number of filter elements each comprising a first and second filter may be arranged in parallel, as in a conventional stacked disc cartridge filter.

The filter devices are useful for treating liquids containing suspended material, particularly biological fluids such as serum, plasma, urine, milk, which may be in diluted or concentrated form. A particular problem addressed by the invention is the removal of mycoplasma from serum. Mycoplasma removal is very difficult because the mycoplasma lack a rigid cell wall and can therefore deform under pressure and squeeze through a conventional polymeric 0.2 micron bacterial membrane. Mycoplasma infection of tissue culture fluids is very serious and costly. In addition, tissue culture fluids are difficult to filter due to their viscosity and particulate content which causes membrane fouling. By use of this invention, these problems can be overcome.

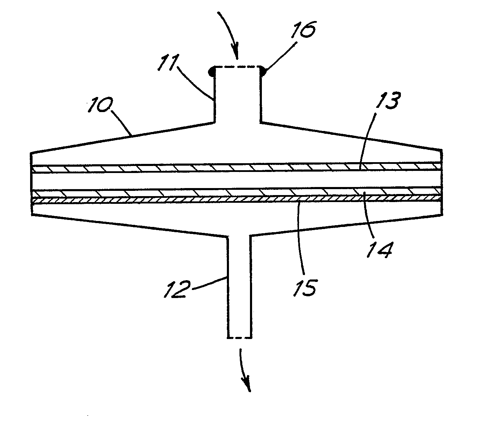

Reference is directed to the accompanying drawing which is an axial section through a filter device according to the invention. The device comprises a housing 10 having an entry port 11 and an exit port 12, the whole defining a passage for fluid. Extending across this passage are two filters in series, a prefilter 13, and an inorganic membrane 14, 15 having a system of larger pores 14 extending in from a first face and interconnecting with the system of smaller pores 15 extending in from the second face. The first face is positioned on the upstream side of this membrane. The housing may be of circular cross-section, or may alternatively be a similar shape such as hexagonal or octagonal. The two filters are sealed into the housing round their periphery by means well known in the art. The entry port 11 is shown having a luer lock 16 for attachment to a syringe.

The following examples illustrate the invention. In these examples, the second filter was an asymmetric anodic aluminium oxide membrane prepared by the technique described in EPA 178831. The pores in the system or larger pores had a diameter of 0.2 microns and the pores in the system of smaller pores had a diameter of 0.1 microns.

Example 1

Neat gamma irradiated foetal calf serum (from a commercial source) was passed through a filter device as illustrated in the drawing, except that a prefilter 13 was absent. The membrane filter blocked after three to four drops (using maximum hand pressure on a 10 ml syringe). This was repeated using a device as above but containing also a binderless glass fibre pre-filter (Whatman GF/F). 8.5 ml of the serum could be passed through the device under the same conditions.

Using 10% serum, which is commonly used in tissue culture media, 120ml could be passed through the device containing the pre-filter compared with 10 ml without the prefilter.

Example 2

Disposable syringe filter devices containing asymmetric anodic membranes without prefilters were compared for membrane orientation using tissue culture broth containing mycoplasma bacteria at a concentration of $6.4 \times 10^4$/ml. The times for 5 ml of this culture to pass through the device under 0.6 MPa were measured. The number of mycoplasma in the filtrate was also measured.

| | Inverted (small pore region downstream) | Non Inverted (small pore region upstream) |
|---|---|---|
| Time (5 ml) | 5 sec. | 165 sec. |
| Efficiency of Removal (%) | 100 | 100 % |

With particulate containing fluids, inverting the membrane and using a prefilter improves the flow rates and increases efficiency at higher challenges (e.g. $10^7$/ml).

Example 3

Disposable syringe filter devices containing asymmetric anodic membranes with the large pore surface facing upstream were compared, with and without prefilters, using tissue culture broth containing Mycoplasma orale at a concentration of $1.3 \times 10^5$/ml. In each experiment, 10 ml of fluid was passed through the filter device. Using devices without a pre-filter, the mycoplasma retention efficiency was 99.96%. Using devices with a pre-filter, the retention deficiency was 99.99%. The use of a pre-filter reduced by a factor of four the number of organisms passing through the device.

**Claims**

1. A micro-filter device comprising a housing having entry and exit ports and defining a passage for fluid and extending across said passage two filters in series, wherein:
- the first filter is a pre-filter
- the second filter is an inorganic membrane having two faces connected by pores, the diameter of the pores adjacent a first face being larger than the diameter of the pores adjacent a second face, the first face being positioned on the upstream side of the membrane.

2. A micro-filter as claimed in claim 1 wherein the second filter is an inorganic membrane having a system of larger pores extending in from a first face and interconnecting with a system of smaller pores extending in from a second face, the first face being positioned on the upstream side of the membrane.

3. A. micro-filter device as claimed in claim 1 or claim 2, where the inorganic membrane is an asymmetric anodic aluminium oxide membrane.

4. A micro-filter device as claimed in any one of claims 1 to 3, wherein the pores in the system of smaller pores of the anodic aluminium oxide membrane have a minimum diameter in the range 5 - 150 nm.

5. A micro-filter device as claimed in claim 4, wherein the minimum diameter is in the range 30-120 nm.

6. A micro-filter device as claimed in any one of claims 1 to 5, wherein the entry port is adapted to receive fluid from a hand-operated syringe.

7. A micro-filter device as claimed in any one of claims 1 to 6, wherein the prefilter is of glass fibre.

8. A method of treating a liquid containing suspended material, which method comprises passing the liquid through the micro-filter device claimed in any one of claims 1 to 7.

9. A method as claimed in claim 8, wherein the liquid is a biological fluid.

10. A method as claimed in claim 9, wherein the biological fluid is serum.

11. A method as claimed in claim 9 or claim 10, wherein the suspended material is mycoplasma.

European Patent Office

# EUROPEAN SEARCH REPORT

Application Number

EP 89 31 0258

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 178 831 (ALCAN)<br>* Claim 1; page 1, lines 5-27 * | 1,2 | B 01 D 29/01<br>B 01 D 63/08<br>A 61 M 1/36 |
| D,A | | 3-5,8-10 | |
| Y | FR-A-2 443 268 (L. LE BOEUF)<br>* Claims; figures * | 1,2 | |
| A | | 8-10 | |
| A | US-A-4 294 594 (JR. SLOANE)<br>* Claims; figures * | 1-11 | |
| A | EP-A-0 083 489 (BRUNSWICK CORP.)<br>* Claims; figures * | 1-11 | |
| A | EP-A-0 123 316 (A/S N. FOSS ELECTRIC)<br>* Abstract * | 1-11 | |
| A | CH-A- 546 090 (WASSER-SAND-FORSCHUNG)<br>* Figure 1 * | 1-11 | |
| A | US-A-4 483 825 (K. FATCHES)<br>* Figures * | 6 | TECHNICAL FIELDS SEARCHED (Int. Cl.5)<br><br>B 01 D<br>A 61 M |
| A | FR-A-2 573 984 (INTERMEDICAT)<br>* Figures; claims * | 6 | |
| A | FR-A-2 419 058 (ASAHI KASEI KOGYO K.K.)<br>* Figures 5,9 * | 6 | |
| A | EP-A-0 155 003 (ASAHI MEDICAL) | | |
| A | DE-A-2 921 924<br>(BIOTEST-SERUM-INSTITUT) | | |

The present search report has been drawn up for all claims

| Place of search | Date of completion of the search | Examiner |
|---|---|---|
| THE HAGUE | 10-01-1990 | KANOLDT W.W. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)